# EUROPEAN PATENT APPLICATION

(11) **EP 4 530 360 A1**
(43) Date of publication of application: **02.04.2025**
(21) Application number: 23200719.5
(22) Date of filing: 29.09.2023
(51) Int. Cl.: C12Q 1/6841, C12P 19/34

(54) **METHOD FOR SPATIAL BARCODING**

(71) Applicant: Miltenyi Biotec B.V. & Co. KG, 51429 Bergisch Gladbach (DE)
(72) Inventor: BOSIO, Andreas, 51429 Bergisch Gladbach (DE); Perbost, Michel, 51429 Bergisch Gladbach (DE); Pinard, Dr., Robert, 51429 Bergisch Gladbach (DE); DeVries, Anthony, 51429 Bergisch Gladbach (DE); Kinkhabwala, Ali, 51429 Bergisch Gladbach (DE)

(57) **Abstract**

One object of the invention was to provide a method for spatial synthesis of oligonucleotides of known sequence (such as barcode sequences) in a biological sample comprising the steps: (A) Providing a biological sample comprising target cells comprising nucleic acids. (B) Identify target cells in said biological sample and assign each target cell a spatial position within said biological sample, thereby creating a map of the target cells (Figure 8) (C) Adding nucleotides comprising a protecting unit at their 3' positions and a template independent transferase to the biological sample, thereby incorporating said nucleotides to each free 3' hydroxyl group of each nucleic acid in the biological sample (D) Synthesizing an oligonucleotide of known sequence comprising the steps: (i) spatially illuminating the position of the target cells with light, thereby inducing cleavage of the protecting unit from the nucleic acids comprised in said target cells and generating free 3' hydroxyl groups at the 3'ends of said nucleic acids (ii) providing nucleotides comprising a protecting unit at their 3' positions and a template independent transferase, thereby incorporating said nucleotide to the free 3' hydroxyl groups of the 3'ends of said nucleic acids. (E) Repeating step D at least 3 times, thereby generating nucleic acids comprising an oligonucleotide of known sequence.

## Description

### Field of the invention

The present invention relates to the field of nucleic acid analysis, barcoding, spatial transcriptomics and next generation sequencing.

### Background of the invention

The analysis of nucleic acids is an important tool in molecular biology. Typical applications are next generation sequencing and spatial transcriptomics. For such applications nucleic acid sequences are analysed and assigned to e.g. specific cell types. Especially in spatial transcriptomics the identification and localization of specific nucleic acids within a tissue is of high interest. Therefore it is essential to assign specific nucleic acids to their cell source and place within the sample. This is usually done by adding specific oligonucleotide sequences (so called barcodes) to nucleic acids from target cells.

The addition of specific oligonucleotide sequences to target nucleic acids is one of the key steps in sequencing applications. These sequences are needed e.g. to distinguish nucleic acids derived from specific samples or cells, to add specific primer binding sites for targeted amplification and/or sequencing.

Several methods to attach oligonucleotides such as barcodes to target nucleic acids are known in the art. Oligonucleotides may be randomly added to isolated nucleic acids from a sample or a single cell during library preparation e.g. by ligating specific adapters or using specific primers. A more specific option may be the use of randomly placed beads with oligonucleotides on the surface with cavities (e.g. like Illumina DNA array). Beads can be decoded before using the array. It guarantees the quality of the codes and provides the knowledge of where each beads is located. Isolated nucleic acids can be added to the wells and a barcode is attached. However the method relies on isolated nucleic acids. The techniques cannot anticipate where the nucleic acid was localized in the tissue. In addition to that they have no flexibility on how many codes are used and where.

One method for spatial barcoding of nucleic acids from tissue samples is provided by 10x genomics. The kit attaches a spatially defined code to mRNA by contacting a tissue with a surface ("visium slide" ) covered with an array of DNA oligonucleotides and capture sequences attached to the substrate or gel. The mRNAs from the tissue diffuse towards the surface and are captured by hybridization to these oligonucleotides, thereby the position of the mRNA is kept in place and can be correlated to the position in the tissue. The mRNAs are then copied into cDNA and a code sequence is attached. However this method has several limitations such as limited capture sequences on the slide, which leads to the problem that not all sequences may be kept in place. In addition to that, the method comprises many different steps which can be a source of potential errors e.g. synthesis of the capture sequences / array and quality control, limited spot size of an oligonucleotide which is limited to the inkjet deposit, diffusion and reaction of the substrate. These steps make the method complex and time consuming.

Another option to attach oligonucleotide sequences to nucleic acids is by direct synthesis within the tissue as disclosed in US11268091B2. The method is based on the cyclic addition of blocked nucleotides followed by chemical unblocking steps. In such a way oligonucleotides of known sequences are directly added to the biomolecules. However the method is not spatially controllable. Therefore the addition of a spatially defined barcode is not possible.

To overcome the current limitations, an easy, fast and controllable method for spatial barcoding of nucleic acids in target cells directly in the tissue is needed. Our inventors surprisingly found a method for spatially controlled oligonucleotide synthesis to nucleic acids comprised in target cells in a biological sample by the use of spatial illumination.

### Brief description of the invention

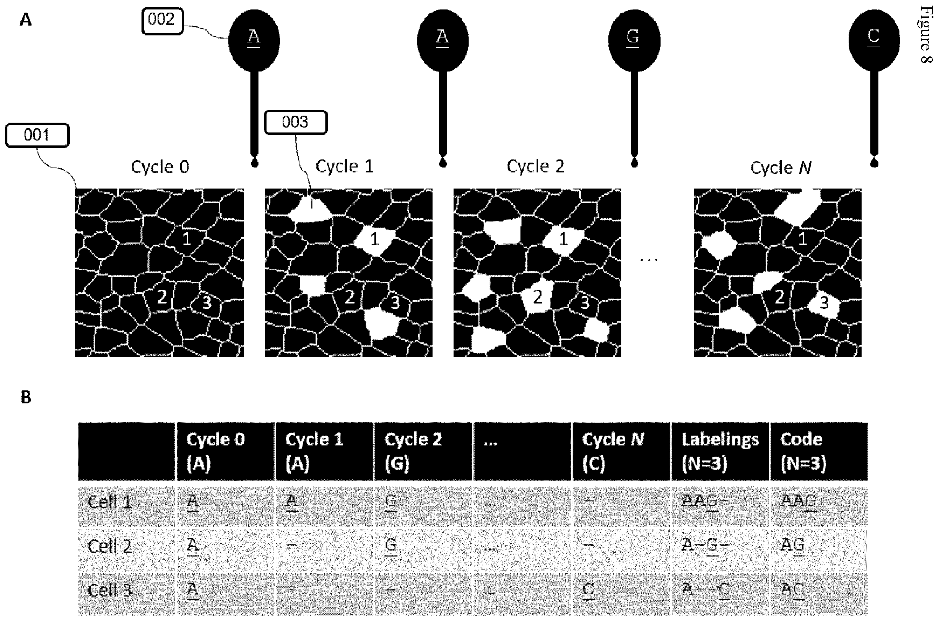

One object of the invention was to provide a method for spatial synthesis of oligonucleotides of known sequence (such as barcode sequences) in a biological sample comprising the steps: (A) Providing a biological sample comprising target cells comprising nucleic acids (especially RNA such as mRNA). (B) Identify target cells in said biological sample and assign each target cell a spatial position within said biological sample, thereby creating a map of the target cells (Figure 8) (C) Adding nucleotides comprising a protecting unit at their 3' positions and a template independent transferase to the biological sample, thereby incorporating said nucleotides to each free 3' hydroxyl group of each nucleic acid (especially RNA such as mRNA) in the biological sample (D) Synthesizing an oligonucleotide of known sequence comprising the steps: (i) spatially illuminating the position of the target cells with light, thereby inducing cleavage of the protecting unit from the nucleic acids (especially RNA such as mRNA) comprised in said target cells and generating free 3' hydroxyl groups at the 3'ends of said nucleic acids (especially RNA such as mRNA) (ii) providing nucleotides comprising a protecting unit at their 3' positions and a template independent transferase, thereby incorporating said nucleotide to the free 3' hydroxyl groups of the 3'ends of said nucleic acids (especially RNA such as mRNA). (E) Repeating step D at least 3 times, thereby generating nucleic acids (especially RNA such as mRNA) comprising an oligonucleotide of known sequence.

In one aspect of the invention the biological sample comprises different target cells having different spatial positions. In each round of synthesis (step Dii) same or different spatial positions of said target cells are illuminated with light. Thereby nucleic acids (especially RNA such as mRNA) comprising different oligonucleotides of known sequence in each target cell are generated (see Figure 8).

### Brief description of the drawings

Figure 1: Potential chemical structures of nucleotides comprising a protection unit. Examples shown for Cytosine triphosphate nucleotides protected in 3' with a photocleavable group
Figure 2: Typical active components of photoresist. 1: benzoin ether; 2: aryl biimidazoles; 3: suitable sensitizer; 4/5:polyfunctional acrylates
Figure 3: Example of chemicals in photoresist which release acid upon irradiation
Figure 4: Example of chemicals in photoresist which release an acid upon irradiation
Figure 5: Examples of chemicals in photoresist which release a sulfonic acid upon irradiation
Figure 6: Examples of chemicals in photoresist which release a base upon irradiation

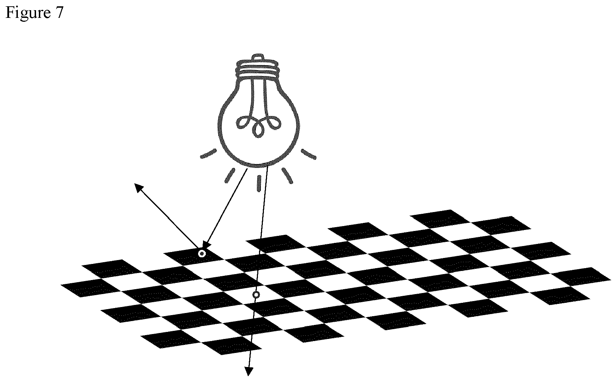

Figure 7: Simple representation of how the IPAM or DMD work using light source and a mask which allow light to go through in some specific location, but prevent it to reach the target in other locations.
Figure 8: Visualization of how the oligonucleotides are synthesized in different cells and not in the other.
Figure 9: Irradiation intensity is highest at the focal plane and lower above and below, as the light cone spreads out away from the focal plane.
Figure 10: A 3' deprotected nucleotide is added to every mRNA with template independent transferase
Figure 11: Irradiation of the photolabile 3' protecting unit releases an hydroxyl group where a new nucleotide can be incorporated
Figure 12: Irradiation of a photosensitive chemical in solution, releases a chemical group capable of cleaving a 3' protecting unit at the 3'end of the mRNA.
Figure 13: A mask, a DMD, and IPAM prevents light to reach part of the tissue, while allowing irradiation of other part. In the irradiated zone the 3' protecting group is removed, while it is not in the non-irradiated area
Figure 14: In the area exposed with light, irradiation of molecule within liquid above the tissue released a chemical which will deprotect the 3' protecting group while it will not release the chemical in the dark zone. Inactive in the figure may be: an enzyme with photoactivable protection, photogenerated acid or base, photoactivated phosphine or photoactivated radical.
Figure 15: A solution of the template independent transferase and one nucleotide comprising a protection unit at the 3' end, here GTP, is added above the tissue to incorporate G. This cannot happen where the previous nucleotide, here A, is still protected
Figure 16: On the right side, after deprotection of the 3' end, a T is incorporated. So a G was incorporated on the previous (figure on the left side), and now a T is incorporated on the right side
Figure 17: After multiple cycles of deprotection and incorporations, different sequences are added at the end of each mRNA of each cell.
Figure 18: An adaptor sequence is added to every mRNA on the tissue. This step does not involve necessary light deprotection after each incorporation.
Figure 19: After synthesis of the code and adaptor sequence, an oligonucleotide, with a sequence complementary to the adaptor sequence is hybridized. It is then elongated with a reverse transcriptase to copy the code and mRNA sequence.
Figure 20: Using a second oligonucleotide with a second adaptor sequence a sequence complementary to part of the mRNA, a PCR reaction amplifies the number of molecules.
Figure 21: After complete synthesis, reverse transcription and PCR, the resulting library should have the following profile: two adaptor sequences, one at each end, a portion of the mRNA sequence and an oligonucleotide of known sequence such as a barcode
Figure 22: The library can be circularized by bringing both ends (3' and 5') near each other, using an oligonucleotide, typically called a bridge. This bridge sequence is complementary to both adaptor 1 and 2.

In the drawings the following reference numbers are used to refer to the following features:
- 001: Image of a tissue segmentation
- 002: Pipetting of a solution containing nucleotides onto the sample
- 003: Illuminated region over a single cell
- 004: Objective lens
- 005: Glass surface where tissue is placed
- 006: Focal plane
- 007: Surface of the tissue
- 008: Tissue slice
- 009: Light cone
- 010: Zone above focal plan
- 011: Focal plan
- 012: In focus illumination of tissue or cell
- 013: Out of focus illumination of tissue or cell, above focus plan
- 014: Out of focus illumination of tissue or cell, below focus plan
- 015: Illumination region, over a single cell or tissue section
- 016: Dark region over a single cell or tissue section
- 017: 3' protected nucleic acid
- 018: Elongated 3'protected nucleic acid
- 019: 3' deprotected nucleic acid
- 020: Elongated 3' deprotected nucleic acid.
- 021: Oligonucleotide complementary to adapter sequence
- 022: Extended oligonucleotide complementary to adapter sequence
- 023: PCR primer ("adaptor 2") complementary to sequence in target nucleic acid and with an extension sequence or sequence complementary to 50% of a bridge oligonucleotide used to circularize the nucleic acid sequence
- 024: Bridge sequence for circularization
- 025: Cell or part of tissue
- 026: Nucleic acid sequence with code sequence and two adaptor sequences (adapter one and two), one at 3' end and the other at 5' end.
- 027: Cell #1
- 028: Cell #2

### Detailed description of the invention

In a first aspect the present invention provides a method for spatial synthesis of oligonucleotides of known sequence in a tissue sample comprising several steps:

### Step A

The method of the invention is for spatial synthesis of oligonucleotides in a biological sample. The biological sample may be a tissue sample. The biological sample may originate from any specimen, like whole animals, organs, organ like structures such as organoids, tissues slices, cell aggregates. The sample may originate from invertebrates, (e.g., Caenorhabditis elegans, Drosophila melanogaster), vertebrates (e.g., Danio rerio, Xenopus laevis) and mammalians (e.g., Mus musculus, Homo sapiens). The biological sample may be or may be derived non-malignant or malignant tissue such as cancerous tissue.

A biological sample may have the form of a tissues slice, cell aggregate or adherent cells. In one embodiment of the invention the sample may have the form of a tissue slice, wherein the tissue slice may have a thickness of up to 20µm, up to 15µm, up to 10µm. In a preferred embodiment the thickness of the tissue slice may be the 8µm. In another preferred embodiment the thickness of the tissue slice may be the 3-4 µm.

A biological sample provided for the method of the current invention may be fixed by a fixative prior to step A. Several fixation methods are known in the art. Most of them are based on the use of reagents such as formalin or aldehyde fixation.

The biological sample may be immobilized by trapping in microcavities or by adherence on top of a transparent substrate e.g. on a glass slide.

The biological sample comprises target cells. Target cells may be a specific cell type such as malignant (such as cancer cells) or non-malignant cells.

Said target cells comprise nucleic acids. The nucleic acids may be deoxyribonucleic acid (DNA) or ribonucleic acid (RNA), preferentially mRNA.

### Step B)

Step B of the invented method comprises the identification of target cells in the biological sample. Each target cell can be assigned to a specific position within the biological sample. Based on that a map of target cells can be created.

Target cells may be identified by methods known in the art. Identification of target cells may be based on morphological features, marker expression profiles or a combination of different features. Morphological features may be identified by microscopy. In another embodiment target cells may be identified by the expression of cell specific markers or proteins. These markers may be identified by fluorescent staining and fluorescence microscopy. Based on such fluorescent microscopy data target cells can be identified and to each target cell a spatial position can be assigned. Based on that a map can be created. Such a map may be created by using segmentation algorithms.

### Step C)

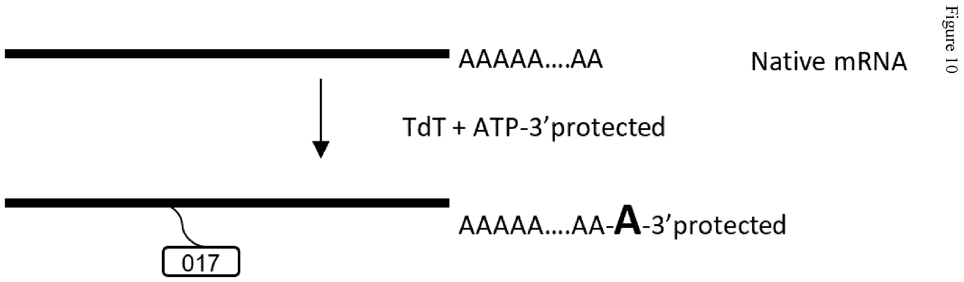

In step C nucleotides comprising a protecting unit at their 3' positions and a template independent transferase are added to the biological sample (Figure 10). The nucleotides comprising a protecting unit at their 3' positions are then incorporated by the transferase to each free 3' hydroxyl group of each nucleic acid (especially RNA such as mRNA) in the biological sample. Nucleic acids (especially RNA such as mRNA) that comprise the nucleotides comprising a protecting unit at their 3'end cannot be extended. No nucleotide can be added enzymatically at this stage, because no free 3'OH groups are available for extension or addition of further nucleotides.

The template independent transferase used for this step is an enzyme and used in aqueous and biologically compatible conditions. In some embodiments, a template-independent polymerase may be a terminal transferase, more preferentially a terminal deoxynucleotidyl transferase (TdT) or a variant thereof. In some embodiments, a template-independent polymerase may be a polymerase modified to become template free.

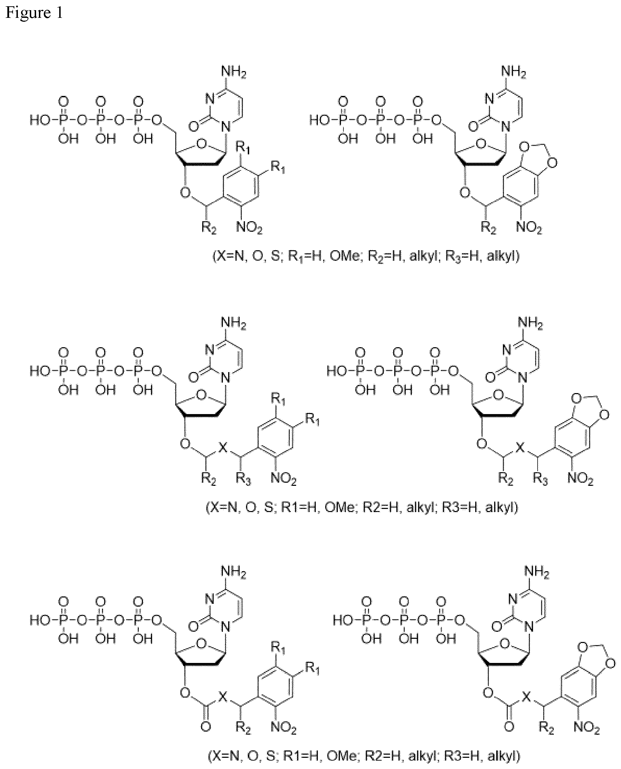

The nucleotides comprising a protecting unit at their 3' positions may be adenine (A), thymine (T), cytosine (C), or guanine (G) or variants thereof (Figure 1).

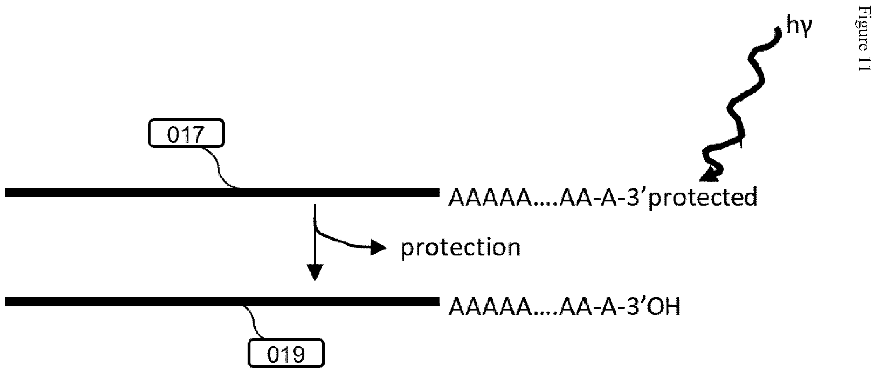

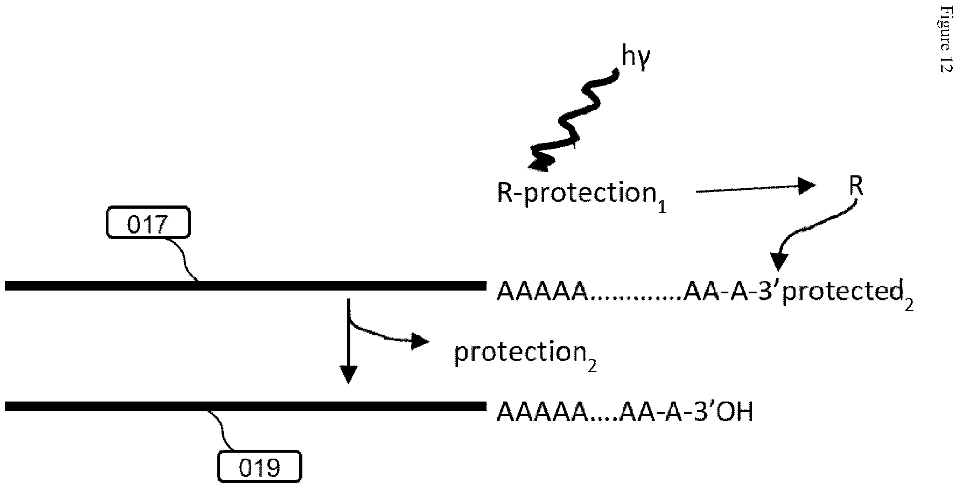

The protection unit comprised at the 3' positions of the nucleotides provided may be directly (Figure 11) or indirectly (Figure 12) cleavable by illumination with light.

Protection units that are directly cleavable with light are photolabile groups such as nitro phenyl groups. Said protection may be photolabile groups such as groups shown in Figure 1.

Moreover protection units may be indirectly cleavable by illumination with light. Exemplary such protection units may be either acid or base labeled molecules such as esters, or cleavable with a phosphine (azido or di-sulfur).

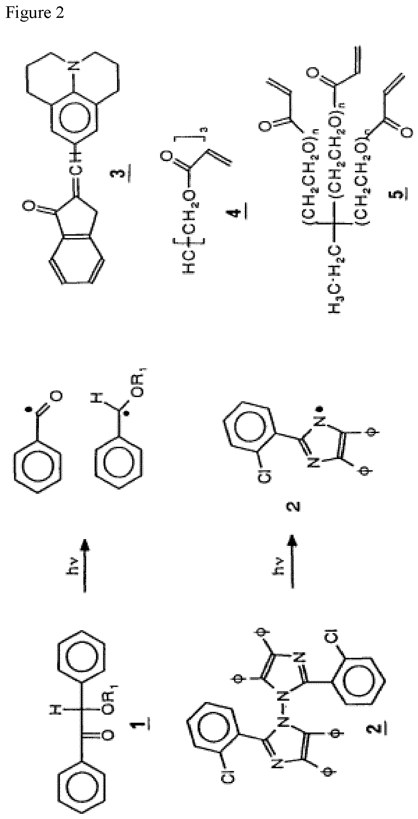

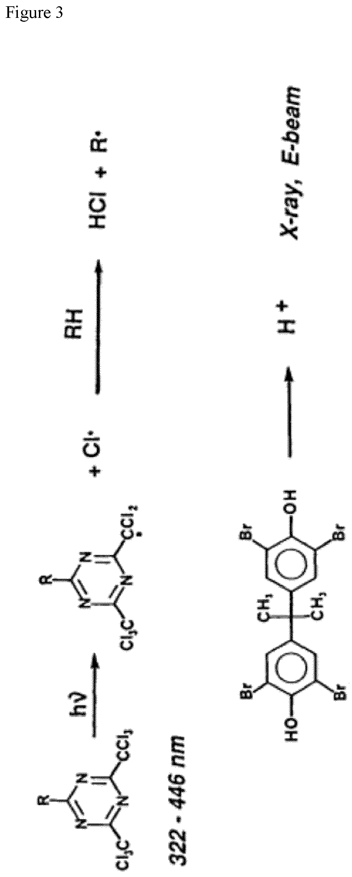

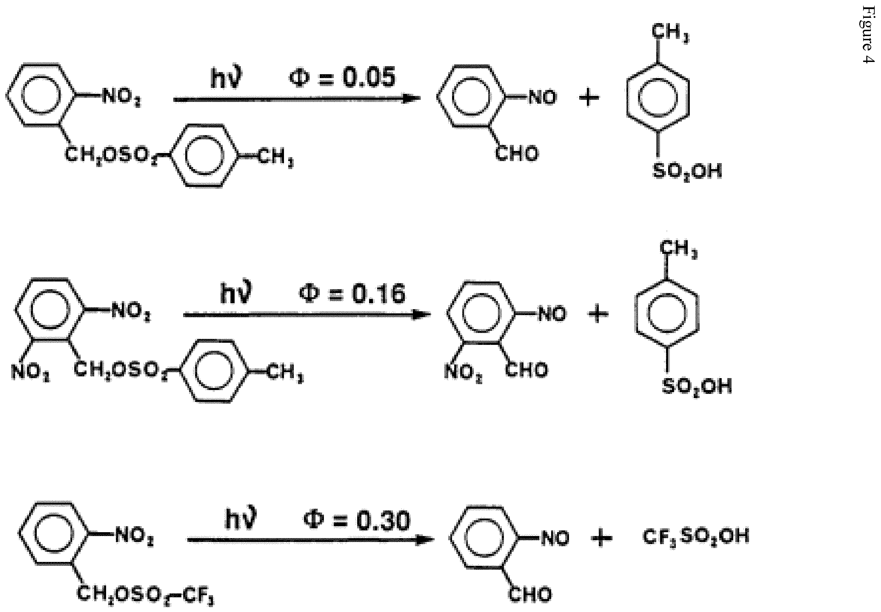

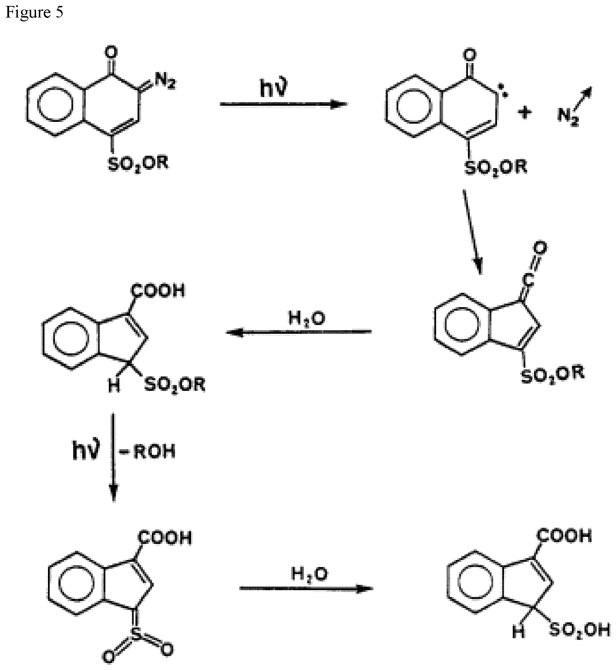

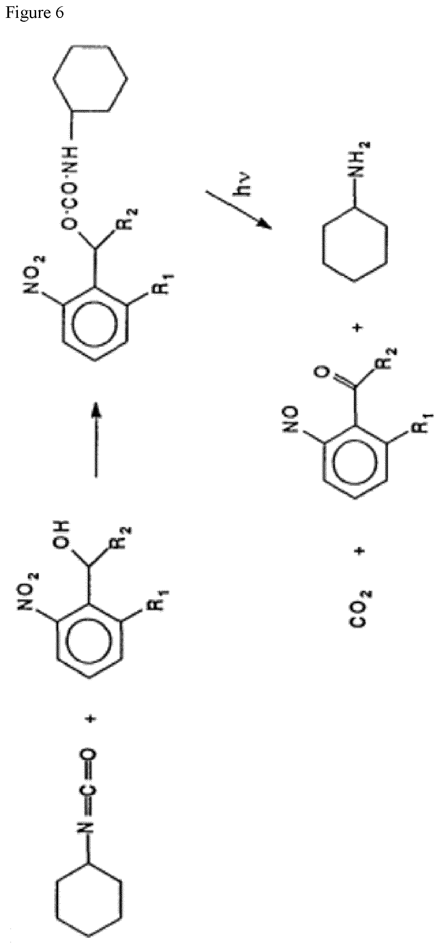

For indirect cleavage with light a reaction partner such as a photo-activatable cleave agent needs to be provided. These photo-activatable cleave agents are capable of removing the protection unit from the incorporated protected nucleotide. Such photo-activatable cleave agents are commonly known in the art. Figure 2-6 exemplary shows such photo-activatable cleave agents and its reactions. Moreover photo-activatable cleave agents are further disclosed in Frechet et al (1992).

### Step D)

Step D describes the synthesis of an oligonucleotide of known sequence. This is based on cyclic removal of the protection unit and the addition of a nucleotides comprising a protecting unit at their 3' positions by a template independent transferase.

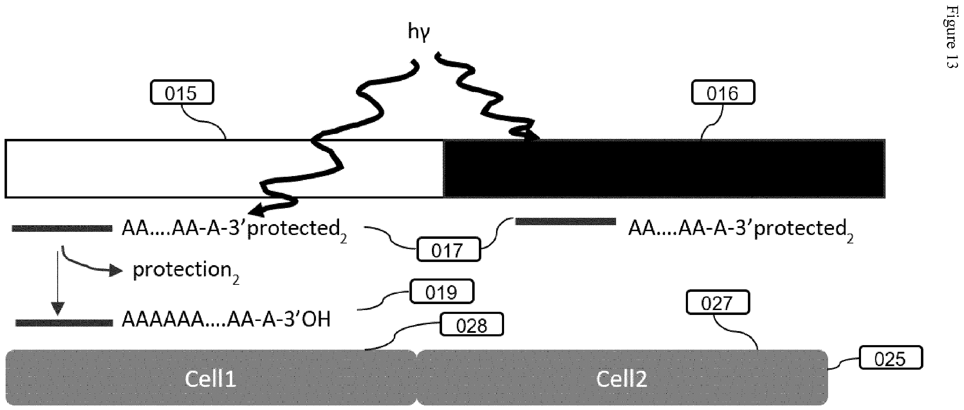

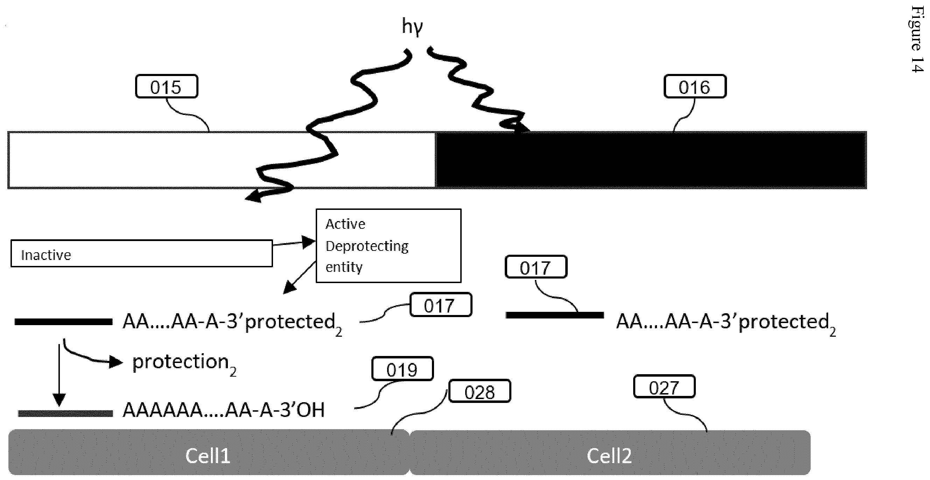

The first step requires the removal of the protection unit from nucleic acids (especially RNA such as mRNA) comprised in the target cells. As described under step C the protection unit can be directly (Figure 13) or indirectly (Figure 14) cleaved by illumination with light.

For indirect cleavage at least one photo-activatable cleave agent capable of removing the protection unit from the incorporated protected nucleotide needs to be added to the biological sample.

Light induced cleavage of the protection unit is done by spatially illuminating the target cells with light. Illumination of target cells can be controlled by e.g. IPAM or other systems such as Digital Micromirror Device (DMD)

In a preferred embodiment a wavelength of 370 nm to 405 nm is used for illumination

Only nucleic acids (especially RNA such as mRNA) comprised in target cells will be illuminated and the protection unit removed only in nucleic acids (especially RNA such as mRNA) of these cells. Therefore, only the nucleic acids (especially RNA such as mRNA) in target cells will have a free 3' hydroxyl group that can be extended by adding a terminal transferase and nucleotides comprising a protecting unit at their 3' positions.

Nucleic acids (especially RNA such as mRNA) in non-illuminated cells will still comprise the protection unit. No nucleotides can be add, because no free 3'hydroxylgroups are available.

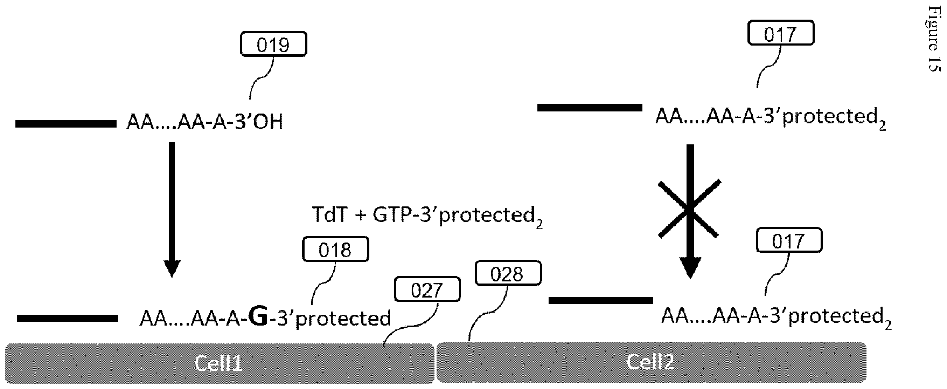

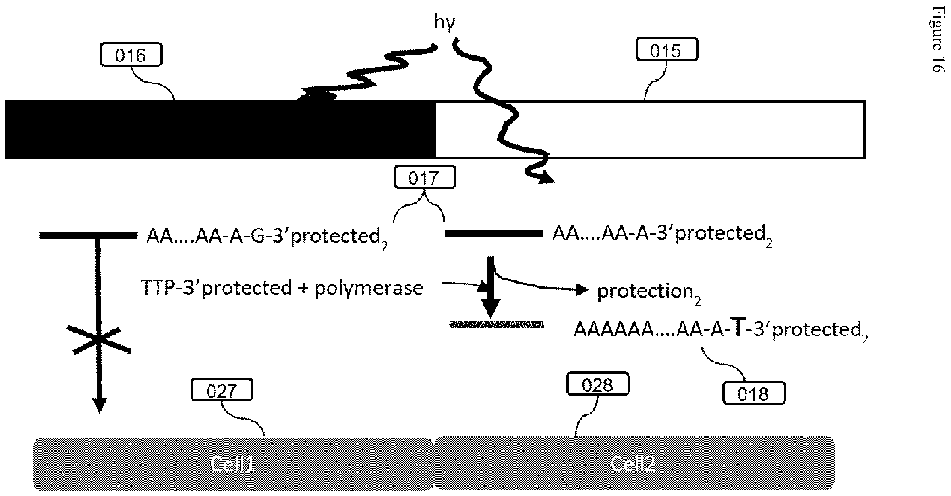

In the next step nucleotides comprising a protecting unit at their 3' positions are incorporated to the free 3' hydroxyl groups of the 3'ends of the nucleic acids(especially RNA such as mRNA) in the target cells by a template independent transferase (Figure 15 and Figure 16). Thereby nucleic acids (especially RNA such as mRNA) are generated that comprise the nucleotides comprising a protecting unit at their 3'end that cannot be extended. No nucleotide can be added enzymatically at this stage, because no free 3'OH groups are available for extension or addition of further nucleotides.

The template independent transferase used for this step is an enzyme and used in aqueous and biologically compatible conditions. In some embodiments, a template-independent polymerase is a terminal transferase more preferentially a terminal deoxynucleotidyl transferase (TdT) or a variant thereof. In some embodiments, a template-independent polymerase may be a polymerase modified to become template free.

The nucleotides comprising a protecting unit at their 3' positions may be adenine (A), thymine (T), cytosine (C), or guanine (G) or variants thereof (Figure 1).

The protection unit comprised at the 3' positions of the nucleotides provided may be directly or indirectly cleavable by illumination with light.

Protection units that are directly cleavable with light are photolabile groups such as nitro phenyl groups. Said protection may be photolabile groups such as groups shown in Figure 1.

Moreover protection units may be indirectly cleavable by illumination with light. Exemplary such protection units may be either acid or base labile molecules such as esters, or cleavable with a phosphine (azido or di-sulfur).

For indirect cleavage with light a reaction partner such as a photo-activatable cleave agent needs to be provided. These photo-activatable cleave agents are capable of removing the protection unit from the incorporated protected nucleotide. Such photo-activatable cleave agents are commonly known in the art. Figure 2-6 exemplary shows such photo-activatable cleave agents and its reactions. Moreover photo-activatable cleave agents are further disclosed in Frechet et al (1992).

The synthesis steps may be repeated at least at least 3 times, 4 times, 12 times or 20 times.

In the invented method, nucleotides comprising a protecting unit at their 3'position are provided subsequently and after step each round of synthesis (step D) the unincorporated nucleotides may be removed from the biological sample. This may be done by standard washing steps.

In each round on synthesis only one kind of nucleotides comprising a 3' protection group is provided. For example in one round of synthesis adenine nucleotides comprising a 3' protection group are provided, only and in another round of synthesis cytosine nucleotides comprising a 3' protection group are provided, only. Based on this it can be controlled which nucleotide is incorporated at which position of the newly synthetized oligonucleotide.

In order to generate specific sequences of oligonucleotides in each round of synthesis the nucleotides comprising a protecting unit at their 3' positions may be same or different, dependent on the sequence that is intended to be generated.

The oligonucleotides of known sequence may be or may comprise barcode sequences, primer binding sites or both.

Optionally after the final round of synthesis the protection unit may be removed from the nucleic acids (especially RNA such as mRNA) by illumination with light.

Nucleic acids (especially RNA such as mRNA) from target cells may be isolated from the biological sample according to methods known in the art

### Oligonucleotides for different target cells

One aspect of the invention is the synthesis of different oligonucleotide sequences to nucleic acids (especially RNA such as mRNA) of different target cells in the biological sample. Thereby the nucleic acids (especially RNA such as mRNA) from each target cell comprise different oligonucleotides of known sequence and the nucleic acid information could be directly assigned to the target cell in the biological sample.

In this aspect of the invention the biological sample comprises different target cells having different spatial positions. In each round of synthesis (step Dii) same or different spatial positions of said target cells are illuminated with light. Thereby nucleic acids (especially RNA such as mRNA) comprising different oligonucleotides of known sequence in each target cell are generated (Figure 8).

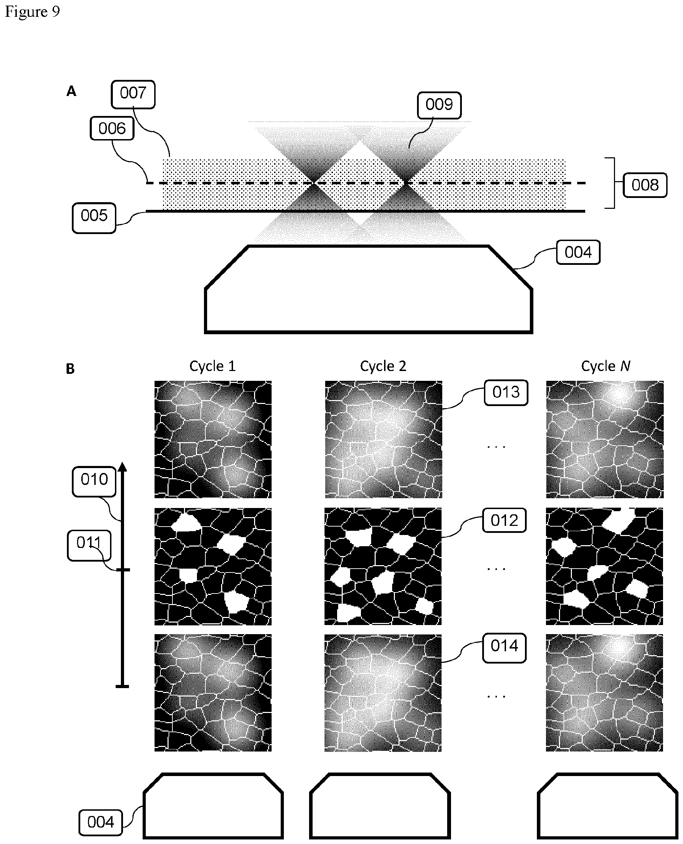

The fidelity of cellular labeling with a specific desired sequence can be affected by various sources of error. A longer-than-expected sequence for a given cell could result from: (1) deprotection of oligonucleotides in non-illuminated regions due to diffusion for the indirect labeling approach, (2) deprotection in off-pixel regions due to background light, (3) deprotection in out-of-focus, laterally displaced regions for sufficiently thick samples illuminated with a high-NA objective (Figure 9). On the other hand, shorter-than-expected cell sequences can result from: (4) insufficient deprotection in the on-pixel regions and (5) transcriptional elongation error (not all sequences extended with a protected nucleotide).

Solution for all sources of error is a long enough sequence for each cell, with a large enough Hamming distance between all cells. In addition to simply increasing the labeling sequence length, an error correcting code strategy could also be employed. The exact library of sequences and labeling strategy would depend on the number of cells to be labeled and the number of cycles to apply.

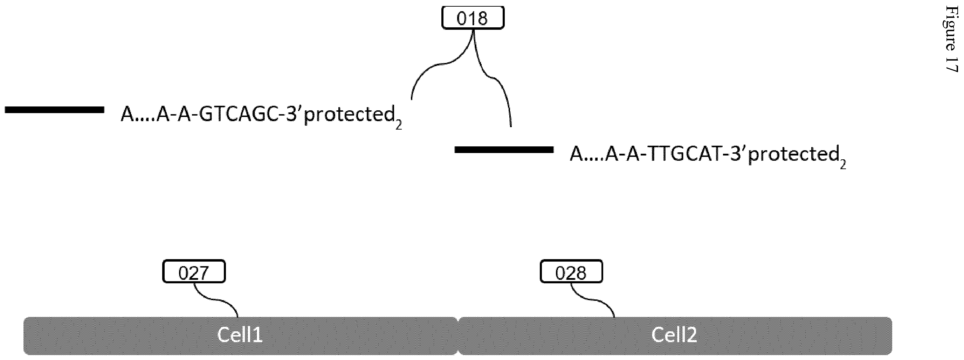

In one embodiment the oligonucleotides may be synthesized in a way that the nucleic acids (especially RNA such as mRNA) of each target cell may comprise an oligonucleotide comprising a specific barcode (Figure 17)

In another embodiment said oligonucleotide of known sequence comprises a barcode and an adapter sequence comprising a primer binding site sequence. The barcode may be different for nucleic acids (especially RNA such as mRNA) of each target cell, while said adapter sequence is same for nucleic acids (especially RNA such as mRNA) of all target cells.

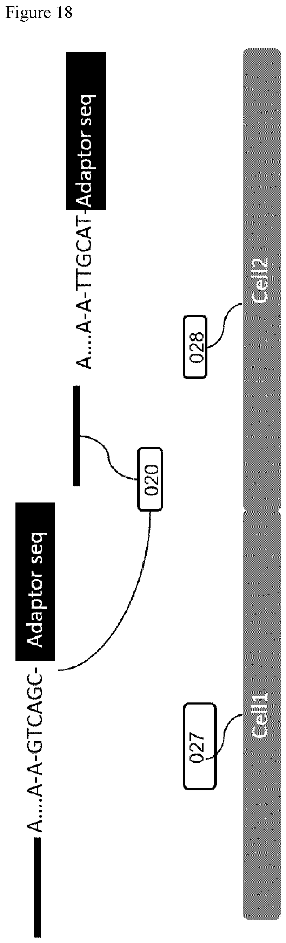

### (see Figure 18)

### Applications

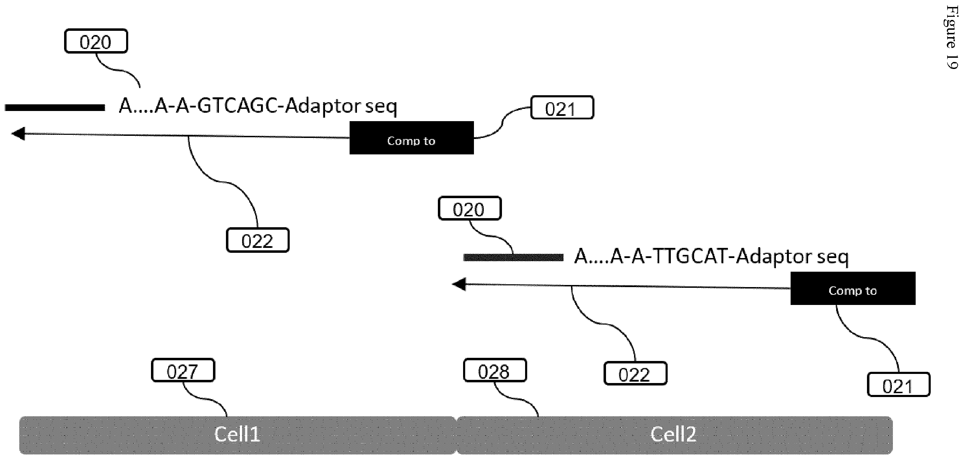

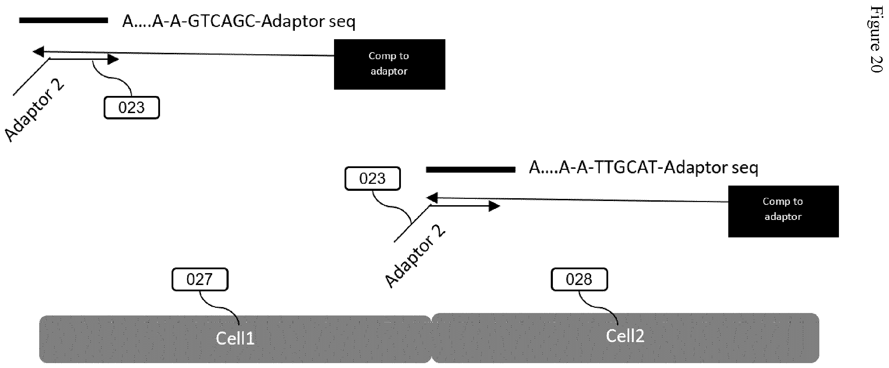

The nucleic acids such as mRNA may be reverse transcribed and amplified (Figure 19 & Figure 20). Finally nucleic acids may be sequenced. Sequences of nucleic acids can be reassigned to the position of the target cell based on the oligonucleotide of known sequence comprising e.g. a barcode sequence. Based on this method sequencing information and spatial tissue information can be correlated with each other.

As mentioned above, the nucleic acids comprising an oligonucleotide of known sequence may be reverse transcribed, amplified and/or sequenced.

For these methods the oligonucleotide of known sequence may comprise an adapter sequence comprising a primer binding site. Said primer binding site may be same or different for nucleic acids from different target cells. Amplification of the nucleic acids may be done by standard polymerase chain reaction (PCR) (Figure 20).

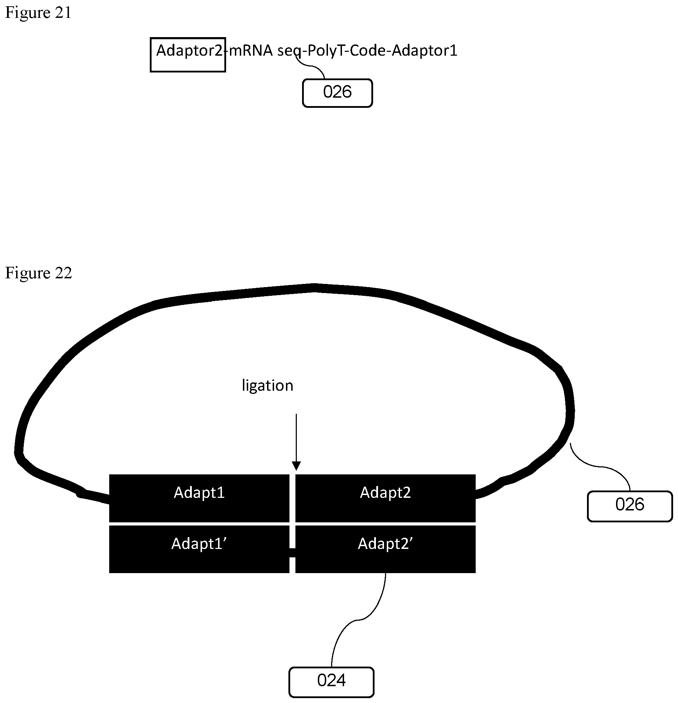

An additional adapter sequence may be incorporated to the 5'end of the nucleic acids by PCR. Such an amplified nucleic acid would comprise a first adapter sequence at the 3'end and a second adapter sequence at the 5' end (Figure 21).

In one embodiment these adapter sequences may be used for circularization of the amplified nucleic acid (as shown in Figure 22).

Here a single oligonucleotide complementary to at least part of the first and second adapter sequence is add to the nucleic acids. Said nucleic acids bind to the complementary sequences and form a circle structure. The 5' and the 3' end of the nucleic acids are brought in close proximity to each other. The ends can be ligated and a circular construct can be formed. Circular constructs may be amplified by rolling circle amplification.

All definitions, characteristics and embodiments defined herein with regard to the first aspect of the invention as disclosed herein also apply mutatis mutandis in the context of the other aspects of the invention as disclosed herein.

### Definitions

Unless defined otherwise, technical and scientific terms used herein have the same meaning as commonly understood by one of ordinary skill in the art to which this invention belongs.

As used herein the term "comprising" or "comprises" is used in reference to compositions, methods, and respective component(s) thereof, that are essential to the method or composition, yet open to the inclusion of unspecified elements, whether essential or not.

The words "binding" and "hybridize" and its grammatical exuviates may be used interchangeably. Hybridization of two nucleic acid strands occurs if they are complementary to each other. Hybridization may occur under conditions known in the art.

As used herein, the term "complementary" refers to the capacity for precise pairing between two nucleotides via Watson and crick base pairing. In explanation, if a nucleotide at a given position of a nucleic acid strand is capable of forming hydrogen bonds with a nucleotide of another nucleic acid strand, then the two nucleic acids are considered to be complementary to one another at that position. Complementarity between two single-stranded nucleic acid molecules may be "partial," in which only some of the nucleotides bind, or it may be complete when total complementarity exists between the single- stranded molecules. A "primer" as used herein is a single stranded oligonucleotide made of nucleotides, which is able to bind to complementary nucleic acid sequences. It is understood that all primers described in the current invention may serve as starting point for nucleic acid synthesis / amplification.

The terms "nucleic acid synthesis" and "nucleic acid amplification" as used herein, can be used interchangeably. The process of nucleic acid synthesis is well known in the art. In Brief: For nucleic acid synthesis a template nucleic acid is provided, which may be single stranded or double stranded. In case double stranded nucleic acid are used initially a first step is the denaturation into single nucleic acid strands (complement and reverse complement) using techniques known in the art. No denaturation step is needed for single stranded nucleic acids. In the next step primer are provided that bind to complementary regions of the nucleic acid strands. The 3'end of the primer is then elongated using a polymerase and a complementary strand is generated by filling with complementary nucleotides. As a result a complementary nucleic acid strand is formed.

The term "reverse transcription" is well known in the art. In brief: First, a primer is hybridized to a RNA molecule. This primer serves as priming site for the synthesis of cDNA by an enzyme with reverse transcriptase activity. The reverse transcriptase can extend the primer and a cDNA is synthetized.

The term "oligonucleotide"; "nucleic acid(s)" and "nucleic acid molecules" refer to biopolymers composed of nucleotide monomers covalently bonded in a chain. An amplified nucleic acid may be named as "amplicon". A nucleic acid may be DNA or RNA.

"Oligonucleotides" according to the current invention may contain all four nucleotides (A, T, G, C) in each position, or only a subset of the four nucleotides in each position. Alternative nucleotides like Uracil, or nucleotide analogues or derivates may also be used.

Oligonucleotides according to the current invention may comprise a barcode sequence. A barcode sequence is a short nucleotide sequence for identification purposes.

### References

Jean M.J. Frechet, Pure & Appl. Chem., Vol 64, No. 9, 1239-1248, 1992
Olejnik J, Lüdemann HC, Krzymanska-Olejnik E, Berkenkamp S, Hillenkamp F, Rothschild KJ. Photocleavable peptide-DNA conjugates: synthesis and applications to DNA analysis using MALDI-MS. Nucleic Acids Res. 1999 Dec 1;27(23):4626-31. doi: 10.1093/nar/27.23.4626. PMID: 10556319; PMCID: PMC148751.
Jerzy Olejnik, Edyta Krzymanska-Olejnik, Kenneth J. Rothschild, Photocleavable Biotin Phosphoramidite for 5' -End-Labeling, Affinity Purification and Phosphorylation of Synthetic Oligonucleotides, Nucleic Acids Research, Volume 24, Issue 2, 1 January 1996, Pages 361-366, https://doi.org/10.1093/nar/24.2.361
https://www.glenresearch.com/reports/gr33-14;

## Claims

1. A method for spatial synthesis of oligonucleotides of known sequence in a biological sample comprising the steps:
A. Providing a biological sample comprising target cells comprising nucleic acids
B. Identify target cells in said biological sample and assign each target cell a spatial position within said biological sample, thereby creating a map of the target cells
C. Adding nucleotides comprising a protecting unit at their 3' positions and a template independent transferase to the biological sample, thereby incorporating said nucleotides to each free 3' hydroxyl group of each nucleic acid in the biological sample,
D. Synthesizing an oligonucleotide of known sequence comprising the steps
I. Spatially illuminating the position of the target cells with light, thereby inducing cleavage of the protecting unit from the nucleic acids comprised in said target cells and generating free 3' hydroxyl groups at the 3'ends of said nucleic acids
II. Providing a nucleotides comprising a protecting unit at their 3' positions and a template independent transferase, thereby incorporating said nucleotide to the free 3' hydroxyl groups of the 3'ends of said nucleic acids
E. Repeating step D at least 3 times, thereby generating nucleic acids comprising an oligonucleotide of known sequence

2. A method according to claim 1, wherein said protecting unit is cleavable by illumination with light

3. A method according to claim 1, wherein in step DII, additionally at least one photo-activatable cleave agent capable of removing the protection unit from the incorporated protected nucleotide is added to the biological sample.

4. A method according to any of the claims 1-3, wherein the nucleotides provided in step C and D are selected from the group of A, T, G, C and variants thereof

5. A method according to any of the claims 1-4, wherein in each round of synthesis same or different nucleotides are provided

6. A method according to any of the claims 1-5, wherein said nucleotides are provided subsequently and wherein after step D), the unincorporated nucleotides are removed from the biological sample.

7. A method according to any of the claims 1-6, wherein the oligonucleotide of known sequence comprises a barcode, a primer binding site or both

8. A method according to any of the claims 1-7, wherein the biological sample comprises different target cells having different spatial positions and wherein in each round of synthesis (step D ii) same or different spatial positions of said target cells are illuminated with light, thereby generating nucleic acids comprising different oligonucleotides of known sequence in each target cell.

9. A method according to any of the claims 1-8, wherein said oligonucleotide of known sequence comprises a barcode and an adapter sequence, wherein said barcode is different and wherein said adapter sequence is same for nucleic acids of all target cells

10. A method according to any of the claims 1-9, wherein illumination with light is done using a wavelength of 370 nm to 400 nm.
